Europäisches Patentamt

European Patent Office

Office européen des brevets

(19)

(11) Publication number: **0 183 404**
A2

(12) **EUROPEAN PATENT APPLICATION**

(21) Application number: 85308026.5

(22) Date of filing: 05.11.85

(51) Int. Cl.⁴: **G 01 N 33/74**

(30) Priority: 19.11.84 IL 73544

(43) Date of publication of application:
04.06.86 Bulletin 86/23

(84) Designated Contracting States:
AT BE CH DE FR GB IT LI LU NL SE

(71) Applicant: YISSUM RESEARCH DEVELOPMENT
COMPANY OF THE HEBREW UNIVERSITY OF
JERUSALEM

Jerusalem(IL)

(72) Inventor: Kaempfer, Raymond Otto Richard
18 Shannan Street
Jerusalem(IL)

(72) Inventor: Rosenthal, Arnon
21 Hamatmid Street
Ramat-Gan(IL)

(72) Inventor: Manor, Daphna
62/10 Ben Sakay Street
Jerusalem(IL)

(72) Inventor: Marsh, Susan
1A Yair Katz Street
Mercaz Carmel Haifa(IL)

(74) Representative: Mair, Richard Douglas et al,
F.J. Cleveland & Company 40-43 Chancery Lane
London WC2A 1JQ(GB)

(54) In-vitro bioassay of erythropoietin.

(57) An *in vitro* boiassay for erythropoietin comprising the steps of preparing a liquid assay mixture from a Manor Cell Preparation incubating the assay mixture in the presence of labeled thymidine to produce a liquid incubation mixture. At the end of an incubation period the cell material is separated from the liquid incubation mixture and the separated cell material is subjected to a quantitative determination of the incorporated labeled thymidine which is proportional to the erythropoietin content of the sample.

The Manor Cell Preparation is prepared by inducing anemia in a rabbit, administering actinomycin D to the anemic rabbit and thereafter collecting bone marrow from the rabbit.

FIG.1

0183404

IN-VITRO BIOASSAY OF ERYTHROPOIETIN

The present invention concerns a novel in vitro bioassay for erythropoietin suitable for clinical application.

The hormone erythropoetin (EPO) plays an essential role in red cell development. As outlined by P. Cotes et al in "Hormones in Blood" edited by C.H. Gray and V.H.T. James, Academic Press New York and London, 1979, page 473, this hormone triggers the proliferation of erythroid precursors in bone marrow and is also required in subsequent stages of erythropoiesis. For these reasons any anomalies in the level of erythropoietin are significant diagnostic indicators and therefore a convenient determination of the erythropoietin level in patients is of major interest in medicine, particularly in the fields of nephrology, haemotology and pediatrics. The clinical usefulness of erythropoietin measurement and its role as a clinical indicator of abnormal erythroid development is outlined by A.J. Erslev in "Kidney Hormones" Vol. 2, edited by J.W. Fisher, Academic Press, New York and London, 1977, page 571.

Several methods for the determination of erythropoietin have been described before. Of the known methods some are in vivo assays and others are in vitro assays. In vivo assays are cumbersome and require large amounts of the material to be tested, properties that restrict the number of samples that can be handled and render them unsuitable as routine diagnostic procedures. Such a method, utilizing mice, is described by Cotes and Bangham in Nature (London) 191,1065 (1961). Another assay utilizing rats is described by Korst et al in J. Lab. Clin. Med. 52,364 (1958).

The known in vitro assays for erythropoietin are more convenient than the in vivo assays, but they too have some limitations which do not allow their employment in routine clinical tests.

One known in vitro method is based on a specific erythroid characteristic which is the inclusion of an iron atom in heme. In accordance with that method, the incorporation of $^{59}$Fe into heme is measured. This method is described, for example, by G. Krystal et al in J. Lab. Clin. Med. 97,144 (1981). This method, however, suffers from a relatively low sensitivity and a lack of specificity. Thus, assays measuring $^{59}$Fe assays are affected by the different levels of iron and transferrin found in sera. Although methods have been devised to correct for these factors, the assay remains too complex for widespread use. Moreover, $^{59}$Fe is a high risk radioactive material which is a further serious limitation.

Another group of known methods is based on bioassays measuring erythroid colony formation as described, for example, by Haga and Falkanger in Blood 53,1172 (1979). Assays measuring erythroid colony formation are more reliable than the assays measuring incorporation of $^{59}$Fe, but they are very laborious and require expertise.

There have also been reported immunoassays of erythropoietin using radio-immunoassay techniques such as by Sherwood and Goldwasser, Blood 54,885 (1979) and Garcia, Sherwood and Goldwasser, Blood Cells 5,405 (1979). However, this method has no potential widespread use, since highly purified erythropoietin is required to ensure the specificity of the antiserum. Alternatively, the use of monoclonal antibodies, such as described by Goldwasser and Sherwood in Br. J. Haematol. 48,359 (1981) and by Lee Huang in Fed. Proc. 41,520 (1981) requires access to hybridoma cell lines that secrete the relevant monoclonal antibodies. Both above requirements present technical and financial barriers for routine use. Another disadvantage in immunoassays is that they are directed against structural rather than biological features of erythropoietin and thus fail to distinguish clinical states where inactive erythropoietin is produced.

Although it has long been realised that determination of erythropoietin in blood is most important for the diagnosis of various diseases, no convenient, clinically applicable method exists for the determination of erythropoietin. It is therefore the object of the present invention to provide a new _in vitro_ bioassay for erythropoietin suitable for clinical application, thereby to fulfill a long felt want.

The invention is based on the realization that a determination of erythropoietin can be based on measurements of DNA synthesis by erythroid precursor cells in the prescence of erythropoietin. However, for a determination of erythropoietin on that basis to be reliable and suitable for clinical applications the following conditions must, among others, be fulfilled:

i      an erythroid precursor substrate must be found whose response to erythropoietin is specific, for example a substrate that is triggered off to synthesize DNA only in the presence of erythropoietin;

ii    the substrate must have a high degree of sensitivity to erythropoietin and respond even to small concentrations thereof;

iii   the response must be highly reproducible;

iv    the substrate must be conveniently available;

v     the substrate must be capable of being stored;

vi    the rate of DNA synthesis by the substrate should be proportional to the concentration of erythropoietin in the test material to be assayed within the range of concentrations that is encountered for clinical diagnosis;

vii   a marker material must be employed that participates exclusively in DNA synthesis;

viii   all operations should be simple and convenient and lend themselves readily for execution by personnel of ordinary skill so that a large number of assays can be performed within a relatively short time.

In principle, cell preparations derived from liver or bone marrow of certain animals respond to erythropoietin to synthesize DNA but the vast majority of such cells do not fulfill the above conditions i - vi and viii. Thus Brandan et al in Br. J. Haematol 48,461 (1981) reported the use of mouse fetal liver cells but in spite of the fact that a lot of effort was devoted in the preparation of fresh cells from embryos at a definite age for each assay, such cells did not respond vigorously to erythropoietin. Bone marrow cells responded even more weakly as reported by Dukes in Ann. NY Acad. Sci. 149,437 (1968). Therefore, none of the methods suggested for the determination of erythropoietin could be applied for routine use.

In accordance with the present invention it has now been found that out of a host of cell preparations capable in principle of synthesizing DNA in response to erythropoietin a particular rabbit bone marrow preparation prepared in a very specific way has all the desired properties. This preparation is obtained by a series of operations comprising:

i     induction of anemia in a rabbit;

ii    administration of actinomycin D to the anemic rabbit; and

iii   thereafter collecting bone marrow from the rabbit.

Such a procedure is described by Manor et al in Exp. Haematol 10,241 (1982). Briefly, such a preparation is obtained by subjecting a rabbit to five consecutive daily subcutaneous injections of 1-acetyl-2-phenylhydrazine in saline which is followed two days later by subcutaneous injection of actinomycin D in saline. Five days later tibiae, femurs and humeri are excised and their bone marrow is collected. After adequate fractionation the cells are suspended in a mixture of α-minimal essential medium (MEM α-medium) and dimethylsulfoxide (DMSO) and gradually cooled down to $-180^{\circ}$C. The so-obtained cell preparation can be stored in a liquid nitrogen freezer for over a year without significant loss of activity and can be withddrawn from cold storage for use at any desired time. In the following specification and claims such a cell preparation will be referred to for short as the "Manor Cell Preparation", it being understood that details in the protocol of Manor et al may be modified as long as the procedure is followed in principle. Also cell preparations obtained by a modification of the procedure of Manor et al will be referred to hereinafter as "Manor Cell Preparation".

Thymidine is a specific precursor of DNA and it has therefore been suggested that DNA synthesis by erythroid precursors can be followed by incorporation of radioactive labeled thymidine which is detectable in the product DNA. (See, for example, Brandan et al, loc. cit.). However, this assay method has so far not found any clinically useful application because of the absence of a suitable substrate.

In accordance with the present invention there is provided an in vitro bioassay for erythropoietin comprising preparing a liquid assay mixture from Manor Cell Preparation, incubating the assay mixture with a sample of the test material to be assayed in the presence of labeled thymidine to produce a liquid incubation mixture, at the end of an incubation period separating the cell material from the liquid incubation mixture and subjecting the separated cell material to quantitative

- 6 -

0183404

determination of labeled thymidine.

The liquid incubation mixture may be used directly without any solid support. In this case separation of the cell material from the liquid component at the end of the incubation period may be effected by filtration, e.g. by means of a cell harvester, and the filter material with the cell material on it may be subjected directly to a radioactivity count.

If desired, the liquid incubation mixture may also be applied to a solid support. In this case the liquid component of the incubation mixture is removed by washing at the end of the incubation period, following which the solid support with the cell material on it may be subjected directly to a count of radioactivity.

The response of the Manor Cell Preparation to erythropoietin is highly specific and the cells synthesize DNA only in the presence of erythropoietin.

It is further an outstanding feature of the assay in accordance with the invention that it is very sensitive. The sensitivity can be expressed in terms of stimulation index which is the ratio between the signal due to the radioactivity incorporated as observed in the presence of erythropoietin and the signal observed in the absence thereof. At an erythropoietin concentration of 50 mU/ml this index usually ranges from 25-40 and these values are much higher than those reported for a $[^3H]$thymidine incorporation assay with mouse fetal liver cells, as reported by Brandan et al loc. cit. where the index of stimulation is less than 4.

As mentioned, a Manor Cell Preparation can be stored in deep freeze and made available whenever required. Thus, a single batch of a Manor Cell Preparation obtained from one rabbit can be frozen in many aliquots that retain their viability and ability to respond to erythropoietin over periods of one year and more. It is estimated that the yield of cells from a single rabbit suffices for over 5,000 triplicate erythropoietin determinations in accordance with the invention.

The assay according to the invention is capable of detecting erythropoietin concentrations as low as 1 mU/ml when the number of the cells in the incubated Manor Cell Preparation ranges between $10^5$ and $10^6$. Best results are obtained when the number of cells is between $10^5$ and $3 \times 10^5$. The response to erythropoietin in terms of incorporated radioactivity is linear within the range from 0-50 mU/ml of erythropoietin and is not saturated before 1 U/ml of the hormone is present.

Where a liquid incubation mixture is used the incubation of the assay mixture with the sample of test material to be assayed and the labeled thymidine can be carried out in any vessel suitable for cell culture. In a preferred embodiment of the invention microtiter plates are used, e.g. sterile plastic molds containing 96 wells each of which serves as an individual vessel for a cell culture. This preferred culture system allows easy handling of many samples including the use of a cell harvester and therefore is convenient and well-suited for clinical use. An added advantage is that small volumes of test sample, ranging between 1-30 µl, are sufficient for reliable measurement.

Any labeled thymidine is suitable for the purpose of the present invention. For example radioactively labeled thymidine may be used and in this case the determination of the labeled thymidine will be by way of radioactivity count to be performed in any adequate manner such as by liquid scintillation spectrometry or by using an antoradiograph. In a preferred embodiment of the invention [$^3$H]thymidine is used having regard to the soft and harmless β-radiation emitted by tritium so that no special protective means against radioactive irradiation are required.

Alternatively the thymidine may be labeled by means of a fluorescent group and the determination is then effected by spectrometric measurements.

It is thus seen that all the requirements for a clinically applicable determination of erythropoietin are fully met in accordance with the present invention.

It has further been found in accordance with the invention that the assay can be effected in the absence of serum in a totally defined culture medium. This novel feature is very attractive when bearing in mind that all known assays for erythropoietin have to be performed in the presence of a serum. The use of a totally defined culture medium lacking serum and not requiring any serum replacing factors is important since it eliminates the background contributed by erythropoietin and by unknown serum-derived growth factors contained in sera. Moreover, the fact that no serum is required is also an important cost saving factor. Iscove et al, in Exp. Cell. Res. 126,121 (1980) developed a method that dispenses with serum for the erythropoietin-dependent colony formation by erythroid precursor cells. However, their system exhibits an essentially absolute requirement for a complex mixture of components containing delipidated albumin, transferrin iron, and sonicated preparations of unsaturated fatty acid, phosphatidyl choline and cholesterol. None of these components are required in accordance with the present invention. Indeed, while albumin, a potential source of contamination with steroid hormones and lower molecular weight proteins, is absolutely necessary in the culture system of Iscove et al it is not required in accordance with the present invention.

The following Examples outline some methods for carrying out the invention thereby illustrating the invention in a non-limiting manner.  In these Examples the following materials were used:

Alpha minimal essentialmedium (MEM $\alpha$-medium) without ribosides and deoxyribosides (Gibco No. 410-200); L-glutamine (Gibco No. 320-5030); 7.5% Na-bicarbonate solution (Gibco No. 670-5080); penicillin-streptomycin solution (Gibco No. 600-5145); fetal bovine serum (Gibco No. 200-6140); gamma globulin (GG)-free fetal bovine serum (Gibco No. 210-6510); methyl cellulose (Fisher Scientific No. M-281); sheep erythropoietin Step III (Connaught Laboratories, Willowdale, Ontario, Canada); bovine serum albumin , Fraction V (Sigma No. A4503); neuraminidase (Behringwerke, Germany No. ORKD 04/05); 2-mercaptoethanol (Fluka, Switzerland); mixed bed ion exchange resin (BioRad No. AG501-X8(d)); dimethylsulfoxide (DMSO) (Fisher Scientific No. D-136); [$^3$H]thymidine, 50-80 Ci/mmole (New England Nuclear No. NET-0272); phytohemagglutinin (PHA) (Difco No. 3110-56-5); 1-acetyl-2-phenylhydrazine (Sigma No. A-4626); actinomycin D (Merck, Sharp and Dohme No. 3298E); [$^{59}$Fe]ferrous citrate, 43 mCi/mg, (New England Nuclear No. NEZ-063); human transferrin (Sigma No. T4515); plastic microtiter plates, 96-well, flat bottom (Linbro No. 76-003-05 or NUNC, Denmark No. 167008); 45-micron filter units (Nalge No. 245-0045); 20- and 45-micron disposable filter assemblies (Gelman Nos. 4192 and 4184 or Schleicher and Schuell, Germany, Nos. FP 030/3 and FP 030/2); disposable microtiter pipettes (Finn-pipette, Finland); disposable 50-ml sterile centrifuge tubes (NUNC, Denmark No. 3-39497); 2-ml screw-capped serum tubes (NUNC, Denmark, No. 1076-1); 10 ml sterile plastic culture tubes (Falcon No. 9219 F22).

Fully iron-saturated human transferrin is prepared as described by Iscove et al, Exp. Cell. Res. 126, 121 (1980), except that the solution is made in MEM α-medium.

EXAMPLE 1

Preparation of highly erythropoietin-responsive red cell
precursors from rabbit's bone marrow

A male rabbit weighing 2.2-2.5 kg is subjected to five consecutive daily subcutaneous injections of 1 ml/kg of 1.25% 1-acetyl-2-phenylhydrazine in saline. Two days after the last injection, 100 µg/kg of actinomycin D in saline is injected subcutaneously. Five days later, tibiae, femurs and humeri are excised and their bone marrow is collected. The bone marrow is placed in a petri dish with 2 ml of MEM α-medium containing antibiotics and is cut with scissors. Twenty ml of the same medium is added and the fragments are dispersed by repeated aspiration into a 35-ml plastic syringe without needle, followed by successive passage of the suspension through 18, 19.5 and 22 guage needles. The cell suspension is passed through sterile gauze wetted in MEM α -medium and washed successively with 25, 15 and 10 ml portions of this medium, transferring the resuspended cells each time to a fresh sterile 50-ml centrifuge tube to eliminate lipid residue. The cells are suspended into 3 ml of MEM α-medium, 20% fetal bovine serum, and the number of viable cells is determined by trypan blue exclusion, using a hemocytometer. To this end, a volume of 5 µl of cells is diluted with 95 µl of MEM α-medium and mixed with 100 µl of a 0.1% trypan blue solution in phosphate-buffered saline, and all viable cells except erythrocytes are counted. The cell concentration is adjusted to $3 \times 10^7$/ml. All preceding operations are carried out at room temperature. The cells are then cooled at $4^{\circ}C$ and an equal volume of cold 20% DMSO in MEM α-medium is added. The cell suspension is distributed into 0.5-ml aliquots in 2-ml screw-cap serum tubes and cooled to $-80^{\circ}C$ in a Planar liquid nitrogen gas-phase programmed freezer at a rate of $1^O$/min. The tubes are then cooled to $-180^O$C with liquid nitrogen and transferred to a liquid nitrogen freezer.

Usually, this procedure yields enough tubes for 50-75 assays of up to 300 wells.

### Example 2

### [$^3$H]thymidine incorporation assay

#### a) Preparation of assay mixture

An aliquot of frozen cells is thawed by placement at $37^O$C. All subsequent operations are performed at room temperature. A 0.5 ml portion of MEM $\alpha$-medium containing 10% fetal bovine serum is added dropwise. The cell suspension is transferred to a 10-ml sterile plastic culture tube and 2 ml of the above medium is added dropwise. After centrifugation for 5 min at 800 rpm, most of the medium is removed by aspiration and the cells are suspended in the remaining medium. A 3 ml volume of the above medium is then added dropwise and the cells are centrifuged. This washing step is repeated once more. Washing the cells is essential to insure an optimal response in the assay. The cell pellet is resuspended into 2 ml of MEM $\alpha$-medium (added dropwise) and a viable count is made as described above. Routinely, over 50% of the cells are viable at this stage.

An assay mixture is prepared, consisting of 1% deionized bovine serum albumin, 7.5-10 µM 2-mercaptoethanol, 0.023% Na-bicarbonate, 1.4 mM L-glutamine, MEM $\alpha$-medium containing 50 units/ml penicillin and 50 µg/ml streptomycin, and 2 x $10^5$ assay cells/ml. These ingredients are mixed thoroughly.

A deionized bovine serum albumin solution is prepared as described by Worton et al in J. Cell. Physiol. 74,171 (1969). An amount of 10 g of bovine serum albumin in 44 ml doubly-distilled water is left overnight at $4^O$C and then stirred twice with 1 g of ion exchange resin for 2 hr, or until the color of the resin has changed. At that point, resin is removed by decantation, an equal volume of MEM α-medium is added, and the solution is filtered through a 45- μm filter and stored at -20°C in aliquots of 3 ml.

b)    Preparation of samples for assay

A volume of 0.6 ml serum, prepared by leaving blood samples 1 h at 4°C and centrifuging for 5 min in an Eppendorf microfuge, is diluted with an equal volume of MEM α-medium and is placed in an Eppendorf heating block at 95°C, or, alternatively, in boiling water.  For human serum, heating for 7 min at 95°C is optimal for removal of inhibitory activity (Manor et al, loc. cit.). For rabbit serum, heating for 2.5 min is sufficient; heating for 7 min causes no further change.  The serum is transferred to a 0°C bath and then centrifuged for 3 min in a microfuge.  The supernatant is used for assay. The one-to-one dilution of serum with medium yields the highest ratio of abnormal to normal human erythropoietin values.

c)    Incubation of samples and assay mixture in microtiter plates

The samples to be assayed and the erythropoietin standard are distributed into the wells of 96-well flat-bottom microtiter plates.  The sample volume can be up to 30 μl.  With human sera, an optimal stimulation index is obtained with 30 μl.  Successive 2-fold dilutions of samples or standard are made in MEM α-medium.  To each well, 100 μl of asssay mixture is dispensed using an eight-channel pipettor (Titerteck, Flow Laboratories).  The microtiter plates are placed at 37°C in water-saturated atmosphere of 5% $CO_2$ in air.

From the cell thawing step up to this point, the procedure takes about two hours.

d)    Incorporation and measurements of [$^3$H]thymidine

After 45 h of incubation, 0.5 µCi of [$^3$H]thymidine is 5 µl of MEM α-medium is added to each well.  After a further incubation for 8 h, the wells are harvested, using a cell harvester (Titertek, Flow Laboratories).  The filter circles are counted in toluene by liquid scintillation spectometry.

<u>e)</u>      <u>Generation of erythropoietin standard curve</u>

The erythropoietin standard (200 U), a lyophilized powder, is dissolved in 10 ml MEM α-medium, passed through a 0.2 mµ filter and stored at -20$^o$C in aliquots of 20 µl or of 1 ml that are subdivided later.  A standard curve is generated by diluting 1 µl of this stock 20-fold in MEM α-medium to give a 1 U/ml solution of which serial two fold dilutions of 20 µl are made subsequently.  Duplicates of 5 µl of each dilution are put into wells and 100 µl/well of assay mixture is added.

<u>Example 3</u>

<u>Colony formation assay for comparison</u>

Assay mixture and samples for assay are prepared exactly as in the [$^3$H]thymidine incorporation assay, except that the assay mixture contains $10^5$ cells/ml, as well as methyl cellulose and fetal bovine serum, selected for its ability to support erythroid colony formation <u>in vitro</u>, to give final concentrations of 0.8% and 30%, respectively, in the assay.  One ml of assay mixture is plated per 35-mm culture dish, containing erythropoietin standard or unknown samples made up to a final volume of 100 µl with MEM α-medium.  Clusters of eight or more cells are counted after 48-72 h as described by Manor et al in Exp. Haematol <u>10</u>, 241 (1982).

<u>Example 4</u>

<u>$^{59}$Fe Incorporation assay for comparison</u>

One µCi of [$^{59}$Fe]ferrous citrate is added to each plate in the above-described colony formation assay.  After incubation for 72 h, the contents of each plate are transferred to a glass tube and washed three times with phosphate-buffered saline. To the cell pellet, one ml of $H_2O$ is added and the suspension is frozen and thawed three times. Heme is extracted as described by De Kerk et al in Blood 52,560 (1978) by the addition of 2 ml Drabkin's solution, 1.5 ml of 0.2 M HCl and 5 ml of ice-cold butane-2-one. After mixing and centrifuging, 2 ml of the top layer are counted directly in a liquid scintillation spectrometer.

Results obtained by following the procedures outlined in these Examples are depicted in the following drawings in which:

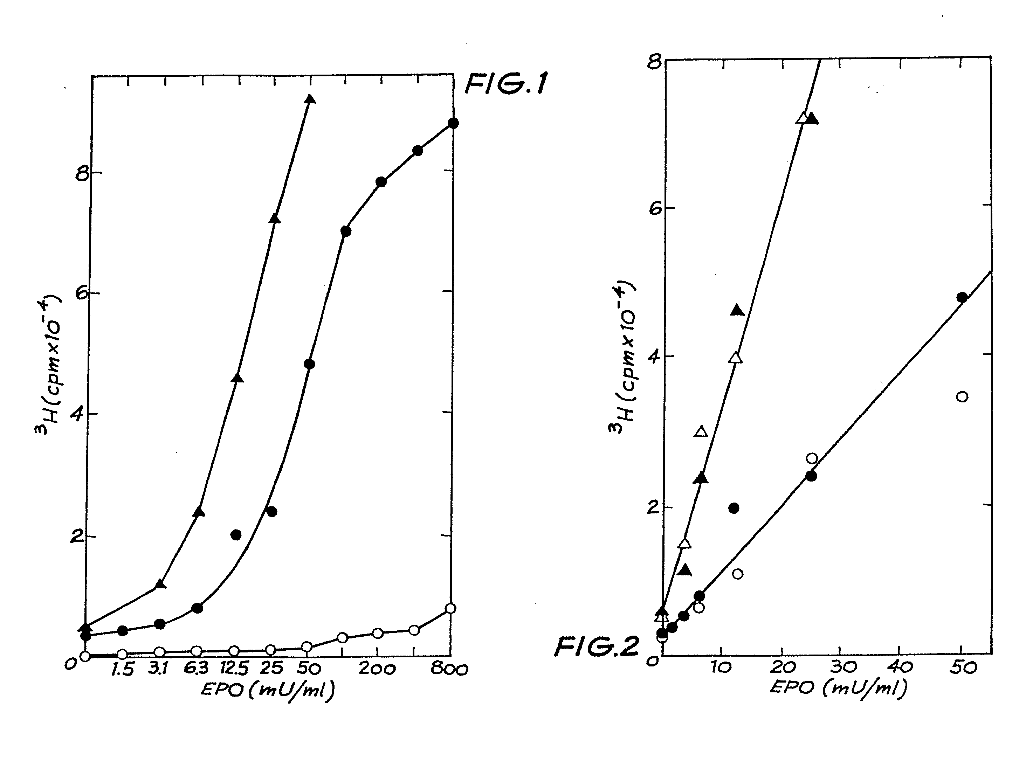

Fig. 1 shows erythropoietin dose-dependence of [$^3$H]thymidine incorporation by rabbit bone marrow cells. The response by cells from two different rabbits treated with phenylhydrazine/actinomycin D (▲ , ●) and from an untreated rabbit (o) is shown. Erythropoietin was present at the indicated concentrations. Each well received 5 µl of erythropoietin and 0.5 µCi of [$^3$H]thymidine. Data shown are the average of triplicate assays. Background was not subtracted.

Fig. 2 shows the linearity of the erythropoietin response in the range of 0-50 mU/ml. [$^3$H]thymidine incorporation data for two different cell preparations shown in Fig. 1 are plotted on a linear erythropoietin scale (▲ , ●). For each of these preparations, two independent assays are illustrated ( ▲, Δ and ●, o, respectively). Each point is the average of triplicate assays.

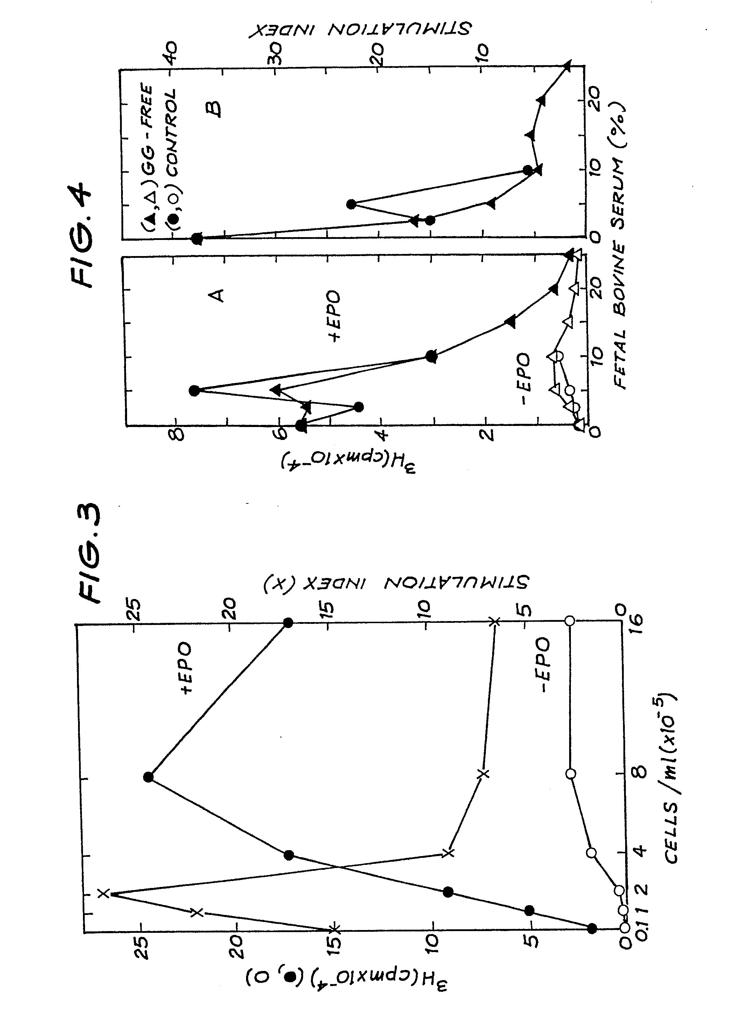

Fig. 3 shows the erythropoietin response as a function of cell number. To assay mixtures, one containing $10^4$ cells/ml and the other $1.2 \times 10^7$ cells/ml, were added to wells in varying proportions to reach the indicated number of cells/ml. The stimulation index represents the ration of [$^3$H]thymidine

incorporated in the presence (●) and absence (o) of 50 mU/ml of erythropoietin.  Data shown are the average of triplicate assays.

Fig. 4 shows the erythropoietin response as a function of fetal bovine serum concentration.  (A) [$^3$H]thymidine incorporation was measured in the presence (▲, ●) or absence (Δ, o) of 50 mU/ml of erythropoietin in assays containing GG-free (▲, Δ) or normal (●, o) fetal bovine serum at the indicated final concentrations (v/v).  Data shown are the average of duplicate assays.  (B), stimulation index calculated from the data of panel (A).

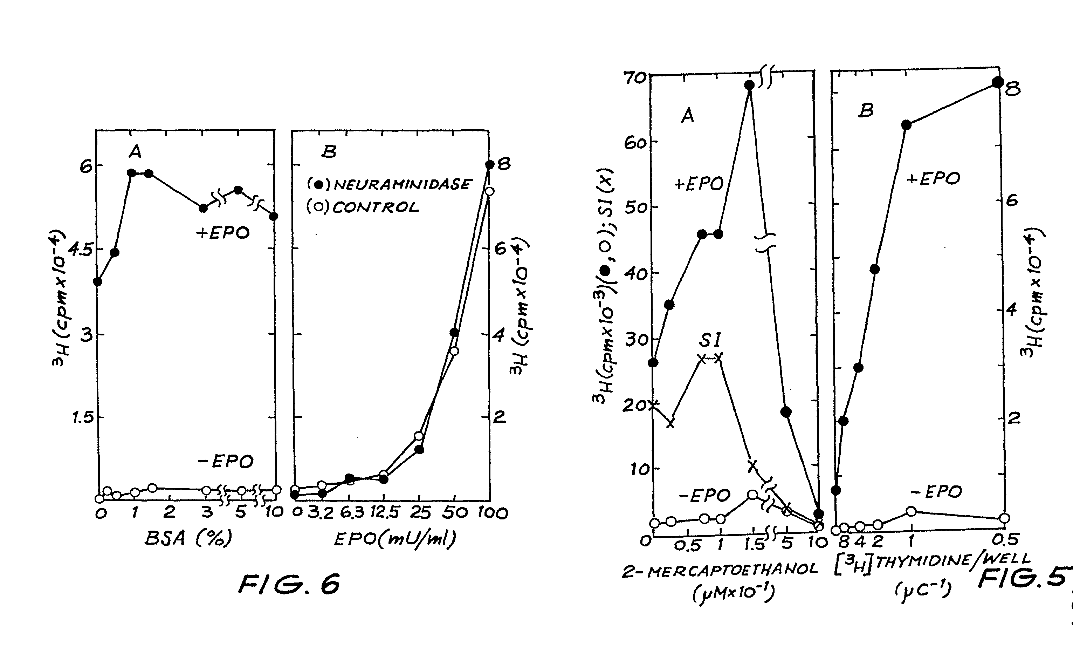

Fig. 5 shows the erythropoietin response as a function of 2-mercaptoethanol or [$^3$H]thymidine concentration.  [$^3$H]thymidine incorporation and stimulation index (SI) were measured in the presence of (●) or absence (o) of 50 mU/ml of erythropoietin in assays containing the indicated final concentrations of 2-mercaptoethanol (A) or [$^3$H]thymidine (B).  Data shown are the average of duplicate assays.

Fig. 6 shows the erythropoietin response as a function of bovine serum albumin concentration (A) or treatment of assay cells with neuraminidase (B).  (A), [$^3$H]thymidine incorporation was measured in the presence (●) or absence (o) of 50 mU/ml of erythropoietin in assays containing the indicated final (w/v) concentrations of deionized bovine serum albumin.  (B), assay cells were thawed, washed, suspended in 0.14 M NaCl, 10 mM Hepes-HCl pH 7.4, and adjusted to 5 x 10$^6$ cells/ml.  The cells were incubated for 10 min at 25$^o$C in the presence (●) or absence (o) of 0.05 U/ml neuraminidase (stock 1 U/ml), diluted with 2 vol fetal bovine serum at 4$^o$C, centrifuged for 5 min at 800 rpm, washed successively with 3-ml portions of 30% and 10% fetal bovine serum in MEM α-medium and taken up in assay mixtures that were plated in wells containing the indicated concentrations of erythropoietin. Data shown are the average of duplicate assays.

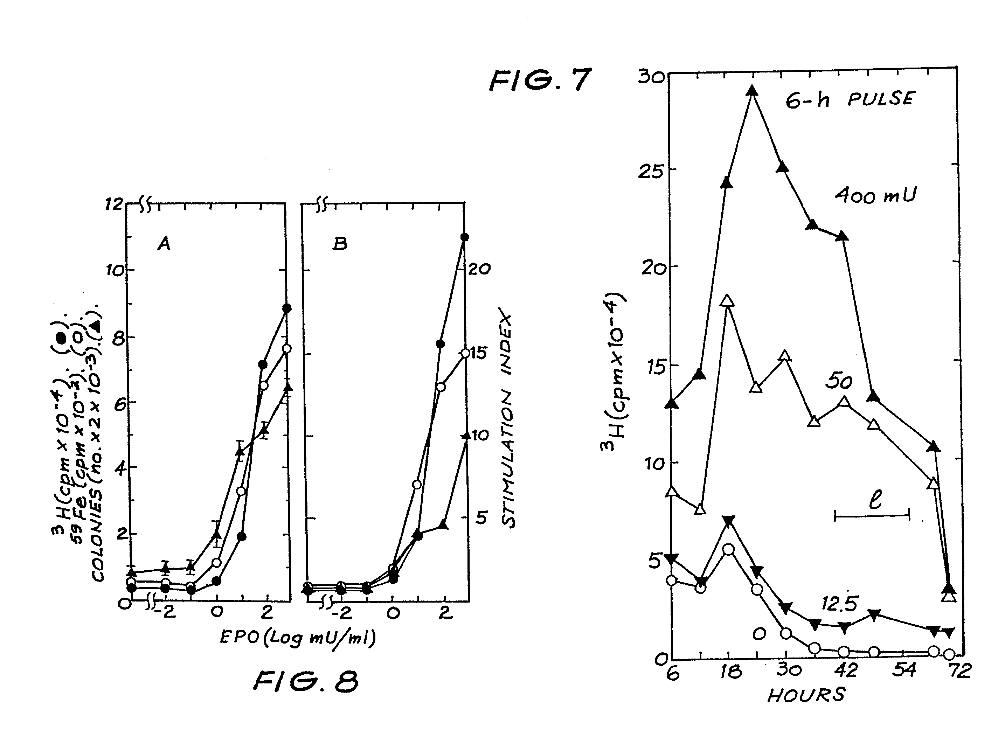

Fig. 7 shows the kinetics of [$^3$H]thymidine incorporation during culture of bone marrow cells as affected by erythropoietin. Assay cells were cultured under standard conditions, in the absence of labeled thymidine. At the indicated times, incorporation of label was measured in individual wells that had received 1 μCi of [$^3$H]thymidine six hours earlier. Erythropoietin was present at 0, 12.5, 50 or 400 mU/ml as noted. Data shown are the average of triplicate assays. $\mathcal{L}$, 40-55 h interval.

Fig. 8 shows a comparison of [$^3$H]thymidine incorporation (●), $^{59}$Fe incorporation (o), and colony formation (▲) assays. (A), data for the three methods with the same preparation of bone marrow cells. The batch of cells used yielded the [$^3$H]thymidine incorporation response depicted by (●) in Fig. 1. Incorporation of $^{59}$Fe was measured in the same plates that were used to study colony formation (see Example 3); data shown are the average of duplicate assays. (B), stimulation index calculated from the data of panel (A).

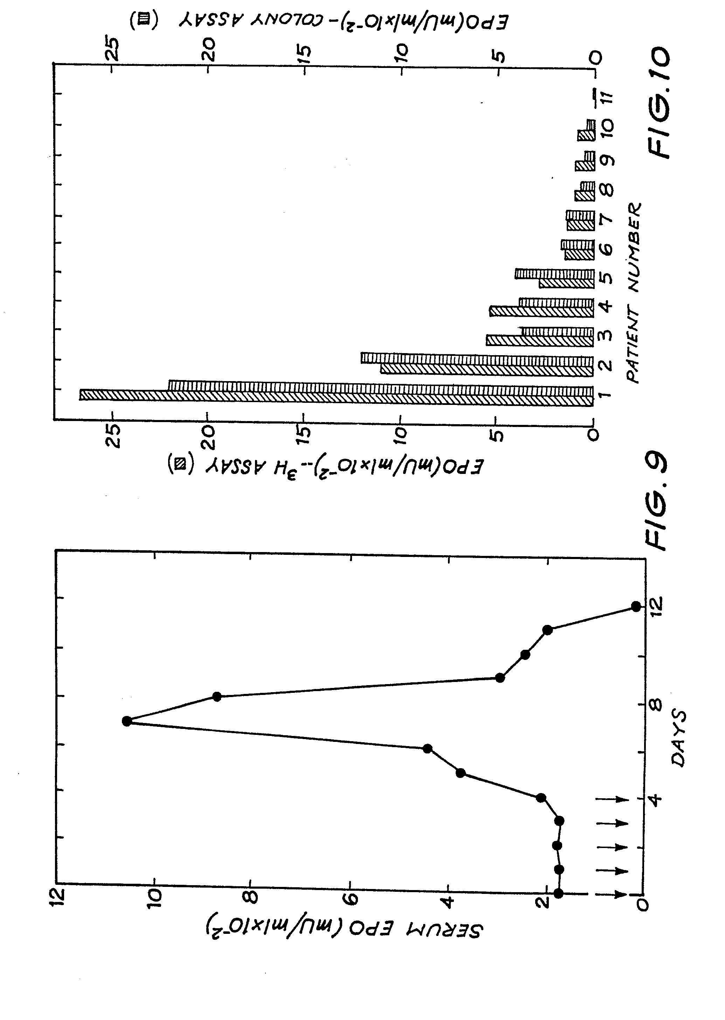

Fig. 9 shows the kinetics of appearance of erythropoietin in rabbit serum upon induction of anemia. A 2.5 kg male rabbit was given five consecutive, daily injections (arrows) of 2.5 ml of 1% 1-acetyl-2-phenylhydrazine. At the indicated times, the erythropoietin titer in 5- μl aliquots of serum was assayed as described in Example 2. Data shown are the average of duplicate assays.

Fig. 10 shows the serum erythropoietin titers for eleven patients. For each serum, erythropoietin was determined both by the [$^3$H]thymidine incorporation assay and for comparison by the colony formation assay.

The following is a brief analysis of the information that emerges from these figures:

Fig. 1 depicts the erythropoietin-dependent incorporation of $[^3H]$thymidine into the DNA of rabbit bone marrow cells.  To enrich such cells for CFU-E (erythroid colony-forming-unit) that is enriching for the capability of repeated cell division and differentiation to red blood cells, the animals were first made anemic by repeated phenylhydrazine injections and then given a single dose of actinomycin D.  Such treatment results in the formation of a high proportion of erythropoietin responders, as shown in the upper two curves which represent cell preparations from two different animals. By contrast, when such treatment is omitted, little if any erythropoietin-dependent stimulation is observed (lower curve).  This inability to respond to erythropoietin is not affected by the inclusion of fetal bovine serum, showing that it is a property of the cells.  As little as 1-1.5 mU/ml of erythropoietin can be detected in the present assay, and concentrations of 10 - 50 mU/ml elicit the incorporation of tens of thousands of cpm.

The incorporation of $^3H$ label is linear with erythropoietin concentration in the range from zero to 50 mU/ml (Fig. 2).  The slope of the incorporation response curve tends to vary somewhat for cell preparations from individual rabbits, but is reproducible for a given preparation (Fig. 2).  The two cell preparations that yielded the data of Fig. 2 represent the most active and least active ones, respectively, among six different preparations.

The erythropoietin assay is performed in 96-well microtiter plates, using a cell harvester and scintillation counter.  This method allows the analysis of hundreds of samples on a given day and requires only small volumes of material for assay (1-30 μl). Moreover, few bone marrow cells are needed per well for an optimal response. This point is illustrated in Fig. 3, which depicts the incorporation of [$^3$H]thymidine as a function of cell number. Although isotope incorporation reaches a maximum beyond $4 \times 10^5$ cells/ml, the optimal stimulation index is observed at $2 \times 10^5$ cells/ml. This value represents an amount of $2 \times 10^4$ cells per well. Under optimal assay conditions, as seen below, a stimulation index of 25- to 40-fold is reproducibly observed in response to 5 mU per well of erythropoietin (50 mU/ml).

The erythropoietin-dependent incorporation of [$^3$H]thymidine is not stimulated significantly by fetal bovine serum, whether or not it is freed of immunoglobins (GG) (Fig. 4A). Indeed, the best index of stimulation is observed in the absence of serum of any kind (Fig. 4B). Actually, several batches of fetal bovine serum were found to inhibit the assay severely, even at 2.5%. The fact that fetal bovine serum is dispensable eliminates the screening of serum batches and offers several other advantages as discussed above.

The known fact as disclosed by Krystal et al. J. Lab. Clin. Med. 97,144 (1981) that 2-mercaptoethanol potentiates the erythropoietin response is confirmed by the experiment of Fig. 5A, which shows that the optimal stimulation index (SI) is attained at concentrations of 7.5 - 10 μM 2-mercaptoethanol.

Fig. 5B illustrates the erythropoietin response as a function of the amount of $^3$H-labeled thymidine. Although isotope incorporation increases up to 2 μCi/well, an amount of 0.5 μCi/well can be used routinely to elicit the response depicted in the previous figures, and gives an optimal stimulation index.

As previously demonstrated by Iscove et al. in (loc. cit.), bovine serum albumin tends to enhance the response to erythropoietin (Fig. 6A), a concentration of 1% being optimal. It is clear, however, that bovine serum albumin stimulates the assay only marginally and can be omitted altogether. We have not observed sensitivity of the rabbit bone marrow assay cells to particular batches of bovine serum albumin, as is often the case for human cells.

It has been reported by Rowley et al. in Blood 57,483 (1981) that erythropoietin-dependent colony formation by human fetal liver or bone marrow cells can be stimulated up to two-fold by treating the assay cells with neuraminidase. However, when examined in the assay system of the present invention, such treatment did not have any perceptible effect on the erythropoietin response (Fig. 6B).

To study the rate of DNA synthesis in erythroid percursors as a function of time in culture and the effect of erythropoietin thereon, assay cells were pulsed for successive 6-h intervals with [3H]thymidine and the incorporation of label into DNA was determined. As seen in Fig. 7, in the absence of added erythropoietin the incorporation of label rises during the 12-18 h interval and then decreases progressively, with little DNA synthesis remaining by 36 h of culture. In the presence of increasing amounts of erythropoietin, however, DNA synthesis not only is stimulated progressively over the 12-18 h interval, but continues over increasingly longer periods, albeit at a declining rate. The recommended time span $\mathcal{L}$ for measuring erythropoietin-supported [3H]thymidine incorporation as seen from Fig. 7 is between 40 and 55 h of culture. During that interval, synthesis of DNA is almost totally dependent upon the presence of erythropoietin. This parameter was examined for six different preparations of assay cells and found to be reproducible.

A correlation between erythropoietin-dependent DNA synthesis and cell division by erythroid percursors in the culture system was revealed by direct viable cell count analysis. Thus, wells that received $2 \times 10^4$ cells and no erythropoietin contained by 72 h $4 \times 10^4$ cells, equivalent to one cell division. In the presence of 50 mU/ml of erythropoietin, the cell number increased to $15 \times 10^4$, equivalent to four cell divisions. After cultivation with 400 mU/ml of erythropoietin, the number of cells had risen to $30 \times 10^4$, equivalent to about five cell divisions. These results correlate well with the data of Fig. 7 and demonstrate the occurrence of erythropoietin-dependent cell division in a completely defined medium.

In Fig. 8, the erythropoietin response of the bone marrow cells used in the assay mixture of the [$^3$H]thymidine incorporation assay is compared with two other methods: incorporation of $^{59}$Fe and colony formation. It may be seen that all three types of assay give a remarkably similar response over an erythropoietin concentration range spanning four orders of magnitude (Fig. 8A). The stimulation index in the [$^3$H]thymidine incorporation assay is comparable to, or even better than, that observed in the other assay methods (Fig. 8B).

The results of Fig. 8 define erythropoietin activity by multiple criteria: incorporation of $^3$H-thymidine (that is, synthesis of DNA), formation of colonies of erythroid precursor cells, and incorporation of $^{59}$Fe into such cells. These results show that the $^3$H-thymidine incorporation assay is at least as specific for erythropoietin as the other two methods. Indeed, as discussed in the preamble of the specification, the $^{59}$Fe incorporation method is less specific, while the colony formation method is much more laborious.

The specificity of the [$^3$H]thymidine incorporation response to erythropoietin is examined in Table 1. Neither the mitogen phytohemagglutinin nor the immunogen BCG are able to elicit any response in the assay cells, in spite of the fact that it is known that they readily stimulate lymphocytes. Moreover, lymphocyte-conditioned medium, known to contain a variety of lymphokines and growth stimulators such as colony-stimulating factors and T-cell growth factor (interleukin-2), stimulates the cells only marginally beyond the level caused by fetal bovine serum present in this medium. Serum is known, as disclosed by Krystal et al. (loc. cit.) to contain small amounts of erythropoietin. This marginal stimulation disappears after brief heat treatment in conditions that leave erythropoietin activity intact (Table 1). Heat treatment of samples is a standard feature of the assay and serves to make it specific. While iron-transferrin is known to stimulate significantly $^{59}$Fe uptake, it has no effect on the erythropoietin-dependent [$^3$H]thymidine incorporation assay at any concentration tested (Table 1). Even though a demonstration of absolute specificity for erythropoietin would demand an exhaustive screening of potential stimulators, the present data as well as results with human sera, to be detailed below, support the interpretation that the assay is specific for eruthropoietin.

The experiment of Fig. 9 illustrates an application .of the present assay method to determine the kinetics of appearance of erythropoietin in the serum of a rabbit during induced anemia. Upon five consecutive daily .injections with phenylhydrazine (arrows), the erythropoietin titer rises sharply, reaching a peak by days 7 to 8 before declining rapidly. This result shows clearly that the method is capable of measuring physiological changes in serum erythropoietin levels.

Application of the method to the analysis of serum erythropoietin levels in human patients is demonstrated in Fig. 10 or 11 cases whose condition is described in Table 2. It may be seen that the erythropoietin levels in sera from a variety of pathological states, as measured by the ($^3$H)thymidine incorporation assay detailed here, correlate extremely well with the values obtained by the colony formation method.

The clinical application of the method according to the invention is further demonstrated as follows:

Sera from 21 normal patients gave an erythropoietin titer of 10.0 mU/ml±1.15 S.E.M. (quintuplicate determinations; range, 2.2 - 22.1 mU/ml.

Serum from a patient with aplastic anemia gave a titer of 1,189 mU/ml±83 S.E.M. After his hematocrit had risen from 21 to 33.4, this patient's serum titer had fallen to 318 mU/ml±75, while 40 days later it had reached 12.6 mU/ml±1.5. These results show clearly that the present assay is capable of monitoring changes in the erythropoietin level in a given patient. This shows that the assay is suitable for routine clinical use and may greatly aid in the establishment of correlations between erythropoietin levels and disease.

TABLE 1.  Specificity of the erythropoietin response

| Experiment No. | Stimulant | Concentration | $^3$H cpm | Stimulation Index | Normal Stimul Ind |
|---|---|---|---|---|---|
| 1 | None | | 2,686 | - | |
| | EPO | 16.5 mU/ml | 18,903 | 8.1 | |
| | | 33 mU/ml | 22,758 | 9.8 | |
| | PHA | 0.1% | 2,098 | 0.8 | |
| | | 0.2% | 2,210 | 0.8 | |
| | | 0.4% | 1,843 | 0.7 | |
| | BOG | 0.25 µg/ml | 1,554 | 0.6 | |
| | | 0.5 µg/ml | 1,713 | 0.6 | |
| | | 1.0 µg/ml | 2,382 | 0.9 | |
| 2 | None | | 1,255 | - | - |
| | EPO | 12.5 mU/ml | 13,104 | 10.5 | - |
| | | 25 mU/ml | 23,028 | 18.3 | - |
| | Ly-CM | 2.5% | 2,660 | 2.1 | 1.5 |
| | | 5% | 3,500 | 2.8 | 2 |
| | | 10% | 4,460 | 3.6 | 2.5 |
| | Serum (Ly-CM) | 10% | 1,800 | 1.4 | 1 |
| | Ly-CM (heated) | 2.5% | 1,200 | 1 | 0.7 |
| | | 5% | 2,005 | 1.6 | 1.1 |
| | | 10% | 2,016 | 1.6 | 1.1 |
| | Serum (Ly-CM) (heated) | 10% | 1,800 | 1.4 | 1 |
| 3 | None | | 242 | - | |
| | EPO + iron-transferrin | 50 mU/ml | 9,376 | 38.7 | |
| | | 0.1 µg/ml | 7,831 | 32.4 | |
| | | 1 µg/ml | 8,920 | 36.9 | |
| | | 5 µg/ml | 7,644 | 31.6 | |
| | | 50 µg/ml | 8,030 | 33.2 | |
| | | 400 µg/ml | 8,893 | 36.8 | |

Erythropoietin assays were performed in triplicate as described in Example 2. In experiment 1, lyophilized, heat-killed BCG bacilli (a gift of Dr. Adam Bekierkunst) and PHA were present at the indicated (w/v) concentrations. In experiment 2, lymphocyte-conditioned medium (Ly-CM) or serum (Ly-CM) were present at the indicated (v/v) concentrations. Ly-CM is the medium of human tonsil lympyhocytes, cultured for 24 h in the presence of 1% PHA. Serum (Ly-CM) is fetal bovine serum diluted to the equivalent concentration present in lymphocyte medium. Where indicated, samples were heated for 2.5 min. at 95°C. On the right, stimulation index values for Ly-CM are normalized to the value obtained with serum (Ly-CM) and serum (Ly-CM) (heated), respectively. To this end, the values for serum (Ly-CM) are set at 1 and all measured values are divided by 1.4.

Background radioactivity was not subtracted.

Table 2. Sera analyzed for erythropoietin titer.

| Patient No. | Diagnosis |
| --- | --- |
| 1 | Diamond-Blackfan hypoplasia |
| 2 | Aplastic anemia |
| 3 | Aplastic anemia |
| 4 | Anemia |
| 5 | Aplastic anemia |
| 6 | Polycythemia |
| 7 | Stress polycythemia |
| 8 | Stress polycythemia |
| 9 | Myeloproliferative disease |
| 10 | Polycythemia |
| 11 | Polycythemia |

It is thus seen that the assay according to the invention is suitable for the analysis of human serum erythropoietin levels. Sera from a group of normal control patients gave a titer of 10+1.15 mU/ml. the higher levels determined in a number of different patients with aplastic anemia, polycythemia or other disorders (Table 2 and Fig. 10) correlate extremely well with values obtained by the colony formation method. Moreover, the assay was capable of detecting a striking, progressive decrease in erythropoietin level in an aplastic anemic patient that recovered from the disease.

Any bioassay for erythropoietin is sensitive to inhibitors or non-erythropoietin stimulators that may be present in serum. Heat treatment of human serum, as described in this specification, removes the latter completely, but leaves some inhibitory activity that diminishes standard sheep or human erythropoietin activity by up to 30%. It must be pointed out, therefore, that bioassays measure the difference between actual erythropoietin activity and any residual inhibitory activity in human serum; the absolute erythropoietin level remains unknown. In spite of these limitations, the assay described here readily detects changes in erythropoietin level, relative to the normal value, that are associated with stress or pathology and as such is a useful indicator of clinical changes.

The invention provides optimal conditions for measuring erythropoietin levels in human serum as those that give the highest stimulation index as well as the highest ratio between abnormal and normal values. The procedures described herein are designed on this basis.

CLAIMS

1.    An in vitro bioassay for erythropoietin comprising preparing a liquid assay mixture from a Manor Cell Preparation, incubating the assay mixture with a sample of the test material to be assayed in the presence of labeled thymidine to produce a liquid incubation mixture, at the end of an incubation period separating the cell material from the liquid incubation mixture and subjecting the separated cell material to a quantitative determination of incorporated labeled thymidine.

2.    A bioassay according to Claim 1, wherein the labeled thymidine is ($^3$H)thymidine, and said determination is by way of radioactivity count.

3.    A bioassay according to any one of Claims 1 to 2, wherein the incubation mixture is left in liquid state.

4.    A bioassay according to any one of Claims 1 to 3, wherein the incubation mixture is applied to a solid support.

5.    A bioassay according to any one of Claims 1 to 3, wherein the aliquots of a liquid Manor Cell Preparation are introduced into the wells of a multi-cell microtiter plate and the liquid in each well is incubated with test material to be assayed in the presence of radioactively labeled thymidine.

FIG.1

FIG.2

1/5

0183404

FIG.3

FIG.4

0183404

FIG. 6

FIG. 5

FIG.7

FIG.8

4/5

0183404

FIG.9

FIG.10

5/5

0183404